# EUROPEAN PATENT APPLICATION

(11) **EP 4 209 173 A1**
(43) Date of publication of application: **12.07.2023**
(21) Application number: 21864389.8
(22) Date of filing: 02.09.2021
(51) Int. Cl.: A61B 5/02, A61B 5/022, A61B 5/145, A61B 5/1455

(54) **BIOLOGICAL INFORMATION CALCULATION SYSTEM**

(30) Priority: 03.09.2020 JP 2020148047; 03.09.2020 JP 2020148048; 25.09.2020 JP 2020160846
(71) Applicant: SSST Co., LTD., Ueda-shi, Nagano 386-0017 (JP)
(72) Inventor: ISHIZAWA, Hiroaki, Ueda-shi, Nagano 386-8567 (JP); KURASAWA, Shintaro, Ueda-shi, Nagano 386-0017 (JP); CHINO, Shun, Ueda-shi, Nagano 386-0017 (JP)
(74) Representative: Plasseraud IP
(86) International application number: PCT/JP2021/032232
(87) International publication number: WO 2022/050334

(57) **Abstract**

[Problem]

Provided is a biometric information computing system that attempts improvement of evaluation accuracy.

[Solution]

The biometric information computing system that outputs information relating to a feature of blood carbon dioxide of a user includes obtaining means, a database, and generating means. The obtaining means obtains evaluation data based on a pulse wave of the user. The database stores classification information generated using a plurality of training data, and the training data is a pair of input data based on a preliminarily obtained training pulse wave and reference data including the feature of blood carbon dioxide associated with the input data. The generating means refers to the database and generates an evaluation result including the feature of blood carbon dioxide for the evaluation data.

## Description

### TECHNICAL FIELD

The present invention relates to a biometric information computing system that outputs information relating to a feature of blood carbon dioxide of a user.

### BACKGROUND ART

Conventionally, as a method for estimating information relating to a living body, such as a blood carbon dioxide partial pressure (PaCO₂), of a user, for example, a method as disclosed in Patent Document 1 has been proposed.

Patent Document 1 discloses the method that constructs an average frame for each of at least two plethysmograms having mutually different light wavelengths, and then uses it to estimate a gain between the two average frames for these two plethysmograms, and this gain value may be used to estimate information for obtaining blood oxygen saturation or other components present in the blood such as hemoglobin, carbon dioxide or others.
Patent Document 1: JP-T-2020-513876

### DISCLOSURE OF THE INVENTION

### PROBLEMS TO BE SOLVED BY THE INVENTION

Here, when a value, such as a blood carbon dioxide partial pressure, of a user is measured, a health condition of the user can be obtained in detail by monitoring the measurement values at a specific cycle. However, when the measurement values are monitored, reduced evaluation accuracy, due to increased variations of the measurement values caused by, for example, measurement conditions, has been a problem. In this respect, Patent Document 1 estimates a feature of blood carbon dioxide based on a plethysmogram using two types of light wavelengths. Therefore, it is concerned that slight differences between angles of light emission and incidence into a sensor may cause significant variations in measurement values.

Therefore, the present invention has been invented in consideration of the above-described problems and an object of the present invention is to provide a biometric information computing system that attempts improvement in evaluation accuracy.

### SOLUTIONS TO THE PROBLEMS

A biometric information computing system according to a first invention is a biometric information computing system for outputting information relating to a feature of blood carbon dioxide of a user including obtaining means that obtains evaluation data based on a pulse wave of the user, a database that stores classification information generated using a plurality of training data, the training data being a pair of input data based on a preliminarily obtained training pulse wave and reference data including the feature of blood carbon dioxide associated with the input data, and generating means that refers to the database and generates an evaluation result including the feature of blood carbon dioxide for the evaluation data.

In the biometric information computing system according to a second invention, which is in the first invention, the reference data includes state information indicating a health condition of an examinee from whom the training pulse wave has been measured, the classification information includes a trained model generated by machine learning using a plurality of the training data, and the generating means includes referring to the trained model and generating the evaluation result with a health condition result included. The health condition result indicates a health condition of the user associated with the evaluation data.

In the biometric information computing system according to a third invention, which is in the second invention, the plurality of training data includes only the input data and the reference data from when the examinee is healthy, and the health condition result indicates that the health condition of the user associated with the evaluation data is healthy or a state other than healthy.

In the biometric information computing system according to a fourth invention, which is in the first invention, the classification information is a calibration model obtained using a PLS regression analysis with the input data as an explanatory variable and the reference data as an objective variable.

In the biometric information computing system according to a fifth invention, which is in the first invention, the obtaining means includes obtaining preliminary evaluation data different from the evaluation data based on the pulse wave, and the generating means includes referring to the database and generating preliminary evaluation result including biometric information of the user for the preliminary evaluation data.

In the biometric information computing system according to a sixth invention, which is in the fifth invention, the database stores preliminary classification information generated using a plurality of preliminary training data, the preliminary training data being a pair of preliminary input data based on the training pulse wave and preliminary reference data including the biometric information associated with the preliminary input data, and the generating means includes referring to the preliminary classification information and generating the preliminary evaluation result for the preliminary evaluation data.

In the biometric information computing system according to a seventh invention, which is in the first invention, the obtaining means includes obtaining preliminary evaluation data different from the evaluation data based on the pulse wave, the classification information includes a plurality of pieces of attribute-based classification information calculated using the training data different from one another, and the generating means includes: selecting means that refers to the preliminary evaluation data and selects first classification information among the plurality of pieces of attribute-based classification information; and attribute-based generating means that refers to the first classification information and generates the evaluation result for the evaluation data.

In the biometric information computing system according to an eighth invention, which is in the seventh invention, the obtaining means includes: obtaining data corresponding to a velocity pulse wave based on the pulse wave; and obtaining data corresponding to an acceleration pulse wave based on the pulse wave as the preliminary evaluation data.

### EFFECTS OF THE INVENTION

According to the first invention to the eighth invention, the generating means refers to the database and generates the evaluation result including the feature of blood carbon dioxide for the evaluation data. The database stores the classification information calculated using the plurality of training data. Therefore, when the evaluation result is generated, it is possible for the evaluation result to include the quantitative feature of blood carbon dioxide in the light of the data that has resulted in the past (the input data and the feature of blood carbon dioxide). This allows attempting improvement in evaluation accuracy.

In particular, according to the second invention, the generating means can refer to the trained model and generate the evaluation result with including the health condition result associated with the evaluation data. Therefore, the user can be notified of the health condition determined by the feature of blood carbon dioxide, and the health condition can be obtained easily compared with the case where the user is notified only of the feature of blood carbon dioxide. This allows attempting improvement in convenience for the user.

In particular, according to the third invention, the plurality of training data includes only the input data and the reference data from when the examinee is healthy, and the health condition result indicates whether the health condition of the user associated with the evaluation data is healthy or not. Therefore, even when the input data based on the training pulse wave when it is unhealthy and the feature of blood carbon dioxide cannot be prepared, the result indicating the health condition of the user can be generated. This allows attempting further improvement in convenience for the user without increasing the degree of difficulty when the classification information is generated.

In particular, according to the fourth invention, the classification information is the calibration model obtained by using the PLS regression analysis with the input data as the explanatory variable and the reference data as the objective variable. Therefore, compared with the case where the classification information is calculated using machine learning or the like, the number of training data can be significantly reduced and the calibration model can be easily updated. This allows establishing the biometric information computing system and attempting to facilitate the update.

In particular, according to the fifth invention, the generating means refers to the database and generates the preliminary evaluation result including the biometric information of the user for the preliminary evaluation data. Therefore, the preliminary evaluation result generated by a different perspective from the evaluation result can be used, thereby ensuring multifaceted evaluations. Thus, an appropriate evaluation corresponding to the request by the user can be achieved.

In particular, according to the sixth invention, the generating means includes referring to the preliminary classification information and generating the preliminary evaluation result for the preliminary evaluation data. Therefore, the preliminary evaluation result generated by referring to the information different from the evaluation result can be used, thereby ensuring more multifaceted evaluations. Thus, an appropriate evaluation corresponding to the request by the user can be easily achieved.

In particular, according to the seventh invention, the generating means includes the selecting means that refers to the preliminary evaluation data and selects the first classification information and the attribute-based generating means that refers to the first classification information and generates the evaluation result for the evaluation data. Therefore, the optimal attribute classification information to the feature of the pulse wave is selected, and then, the evaluation result for the evaluation data can be generated. This allows attempting the further improvement of the evaluation accuracy.

In particular, according to the eighth invention, the obtaining means obtains the data corresponding to the velocity pulse wave based on the pulse wave as the evaluation data. The obtaining means obtains the data corresponding to the acceleration pulse wave based on the pulse wave as the preliminary evaluation data. Therefore, the attribute classification information can be selected using the acceleration pulse wave that facilitates the classification of the feature of the pulse wave compared with the velocity pulse wave. The evaluation result can also be generated using the velocity pulse wave that facilitates the calculation of the feature of blood carbon dioxide compared with the acceleration pulse wave. This allows attempting the further improvement of the evaluation accuracy.

### BRIEF DESCRIPTION OF THE DRAWINGS

Fig. 1 is a schematic diagram illustrating an example of a biometric information computing system in a first embodiment.
Fig. 2A and Fig. 2B are schematic diagrams illustrating examples of operations of the biometric information computing system in the first embodiment.
Fig. 3A is a schematic diagram illustrating an example of classification information and Fig. 3B and Fig. 3C are schematic diagrams illustrating examples of processes on sensor data.
Fig. 4A is a schematic diagram illustrating an example of a configuration of the biometric information computing device and Fig. 4B is a schematic diagram illustrating an example of a function of the biometric information computing device.
Fig. 5A and Fig. 5B are schematic diagrams illustrating examples of a sensor.
Fig. 6 is a flowchart illustrating an example of an operation of the biometric information computing system in the first embodiment.
Fig. 7 is a schematic diagram illustrating an example of an operation of a biometric information computing system in a second embodiment.
Fig. 8A and Fig. 8B are schematic diagrams illustrating examples of processes to calculate biometric information for sensor data.
Fig. 9 is a schematic diagram illustrating a modification of the operation of the biometric information computing system in the second embodiment.
Fig. 10 is a schematic diagram illustrating an example of an operation of a biometric information computing system in a third embodiment.
Fig. 11 is a schematic diagram illustrating an exemplary classification of data corresponding to an acceleration pulse wave.
Fig. 12 is a schematic diagram illustrating an exemplary classification of data corresponding to a velocity pulse wave.

### DESCRIPTION OF PREFERRED EMBODIMENTS

The following describes examples of a biometric information computing system in the embodiments of the present invention by referring to the drawings.

### (First Embodiment: Biometric Information Computing System 100)

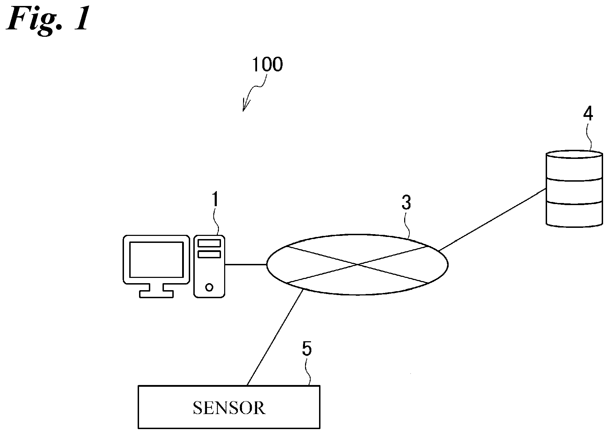

Fig. 1 is a schematic diagram illustrating an example of a biometric information computing system 100 in the first embodiment.

The biometric information computing system 100 is used for outputting information relating to a feature of blood carbon dioxide of a user. The biometric information computing system 100 can generate an evaluation result including the feature of blood carbon dioxide from evaluation data based on a pulse wave of the user. The "feature of blood carbon dioxide" indicates a level of carbon dioxide contained in blood of a user. As the feature of blood carbon dioxide, for example, besides a value of a blood carbon dioxide partial pressure (PaCO₂) being used, a dissolved concentration of blood carbon dioxide and a concentration of bicarbonate (HCO₃⁻) contained in blood may be used or a value considering pH of blood may be used depending on the situation. The following mainly describes the case where the value of the blood carbon dioxide partial pressure is used as the feature of blood carbon dioxide.

For example, as illustrated in Fig. 1, the biometric information computing system 100 includes a biometric information computing device 1, and for example, may include at least any of a sensor 5 and a server 4. The biometric information computing device 1 is connected to the sensor 5 and the server 4 via, for example, a communications network 3.

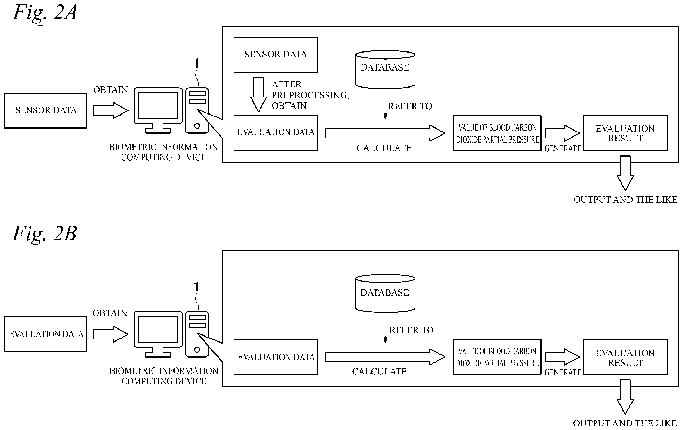

In the biometric information computing system 100, for example, as illustrated in Fig. 2A, the biometric information computing device 1 obtains sensor data generated by the sensor 5 or the like. Afterwards, the biometric information computing device 1 performs preprocessing, such as filter process, on the obtained data, thereby obtaining evaluation data.

The biometric information computing device 1 refers to a database and calculates a feature of blood carbon dioxide (for example, a value of a blood carbon dioxide partial pressure) relative to the evaluation data. The database stores, for example, classification information for calculating the value of the blood carbon dioxide partial pressure from the evaluation data.

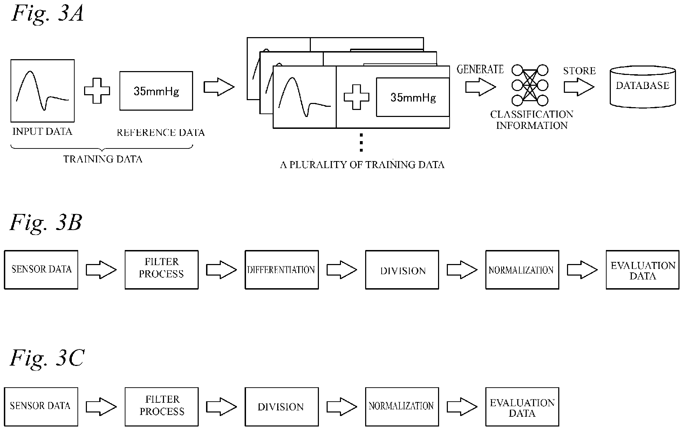

For example, as illustrated in Fig. 3A, the classification information is generated using a plurality of training data, and the training data is a pair of input data based on a training pulse wave that has been obtained in the past and reference data including a value of a blood carbon dioxide partial pressure associated with the input data. Therefore, since data of, for example, examinees whose health conditions and the like are definite can be used as the training data, it is possible to generate reliable classification information compared with the case where simply accumulated data is used. Referring to the classification information can also calculate a quantitative value of the blood carbon dioxide partial pressure without calculation criteria varying over time.

The biometric information computing device 1 generates an evaluation result including the calculated value of the blood carbon dioxide partial pressure and outputs, for example, to a display. The evaluation result may include information indicating an evaluation on the value of the blood carbon dioxide partial pressure, such as "normal" and "abnormal" besides the value of the blood carbon dioxide partial pressure.

As described above, the biometric information computing system 100 according to the embodiment can refer to the database and generate the evaluation result including the feature of blood carbon dioxide for the evaluation data. Therefore, when the evaluation result is generated, it is possible for the evaluation result to include the quantitative feature of blood carbon dioxide in the light of the data that has resulted in the past. This allows attempting improvement in evaluation accuracy.

The biometric information computing system 100 may, for example, as illustrated in Fig. 2B, obtain the evaluation data from the sensor 5 or the like. In this case, the preprocessing to obtain the evaluation data from the sensor data is performed by the sensor 5 or the like.

### <Sensor Data>

The sensor data includes data indicating a feature of pulse wave of a user and may include, for example, data (noise) indicating a feature other than the pulse wave. The sensor data is data indicating an amplitude relative to a measurement period and by performing a filter process depending on the usage and the generation condition of the sensor data, data corresponding to an acceleration pulse wave, a velocity pulse wave, or the like can be obtained from the sensor data.

The sensor data can be generated with a known sensor, such as a strain sensor, a gyro sensor, a photoplethysmogram (PPG), and a sensor pressure sensor. The sensor data may, for example, be an analog signal other than a digital signal. The measurement period when the sensor data is generated is a measurement period of, for example, 1 to 20 cycles of the pulse wave and can be set appropriately depending on the conditions, such as a processing method of the sensor data and a data communication method.

### <Evaluation Data>

The evaluation data indicates data for calculating the feature of blood carbon dioxide. The evaluation data indicates, for example, data corresponding to the acceleration pulse wave based on the pulse wave of the user and indicates an amplitude relative to a specific cycle (for example, one cycle).

The evaluation data is obtained by processing (preprocessing) the sensor data by the biometric information computing device 1 or the like. For example, as illustrated in Fig. 3B and Fig. 3C, the evaluation data can be obtained by performing a plurality of processes on the sensor data. The details of the respective processes will be described later.

### <Database>

The database is mainly used when the feature of blood carbon dioxide relative to the evaluation data is calculated. Besides storing one or more pieces of classification information, the database may, for example, store the plurality of training data used for generating the classification information.

The classification information is a function indicating, for example, a correlation between the preliminarily obtained past evaluation data (input data) and the reference data including the feature of blood carbon dioxide. The classification information indicates a calibration model generated based on a result of an analysis performed by, for example, a regression analysis having the input data as an explanatory variable and the reference data as an objective variable. The classification information can, for example, periodically update the calibration model, and in addition, may generate calibration models corresponding to attribute information, such as a gender, an age, and a health condition of the user.

As a method of the regression analysis used in the generation of the classification information, for example, PLS (Partial Least Squares) regression analysis, a regression analysis using SIMCA (Soft Independent Modeling of Class Analogy) method in which a principal component analysis is performed for each class to obtain a principal component model, or the like can be used.

The classification information may, for example, include a trained model generated by machine learning using the plurality of training data. The trained model indicates, for example, SVM (Support vector machine) and the like besides indicating a neural network model, such as CNN (Convolutional Neural Network) and the like. For the machine learning, for example, deep learning is usable.

For the input data, data of a type the same as that of the evaluation data is used, and, for example, indicates the past evaluation data whose corresponding feature of blood carbon dioxide has been definite. The input data indicates data obtained based on a pulse wave (training pulse wave) of an examinee whose health condition and the like are definite.

For example, the sensor 5 or the like is worn by an examinee, and thus, the sensor data (training sensor data) indicating the feature of training pulse wave is generated. By performing a process on the training sensor data, the input data can be obtained. Besides being obtained from the user of the biometric information computing system 100, the input data may, for example, be obtained from another user than the user. That is, besides being the user of the biometric information computing system 100, the above-described examinee may be those other than the user and may be a large number of specified or unspecified people.

The input data is preferably obtained by the contents similar to, for example, the type of the sensor 5 or the like used when the evaluation data is obtained, the generation condition of the sensor data, and the process condition on the sensor data. For example, by unifying the above-described three contents, the accuracy when the feature of blood carbon dioxide is calculated can be dramatically improved.

The reference data includes the feature of blood carbon dioxide of the examinee measured using a measuring device. For example, when the sensor 5 or the like is worn by the examinee to generate the training sensor data, by measuring the feature of blood carbon dioxide of the examinee, the reference data associated with the input data can be obtained. In this case, while the timing of measuring the feature of blood carbon dioxide is preferably simultaneous with the timing of generating the training sensor data, it may be a timing, for example, before or after approximately 1 to 10 minutes.

As a measuring device, other than, for example, a known device, such as a transcutaneous blood gas monitor TCM5 (manufactured by Radiometer Basel AG) being used, for example, a known device that measures or estimates a blood carbon dioxide partial pressure (PaCO₂) may be used. For example, as a measuring device, a known measuring device that can measure or estimate a concentration of bicarbonate (HCO₃⁻) contained in blood and pH in blood may be used. Besides including the feature of blood carbon dioxide, such as the value of the blood carbon dioxide partial pressure, the reference data may include a measurement value that may be related to the feature of blood carbon dioxide, such as a value of the oxygen saturation and a value of the atmospheric pressure.

### <Biometric Information Computing Device 1>

The biometric information computing device 1 indicates, for example, an electronic device such as a personal computer (PC), a mobile phone, a smartphone, a tablet terminal, and a wearable device, and indicates, for example, an electronic device communicatable via the communications network 3 based on the operation of the user. The biometric information computing device 1 may include the sensor 5. The following describes an example of a case where a PC is used as the biometric information computing device 1.

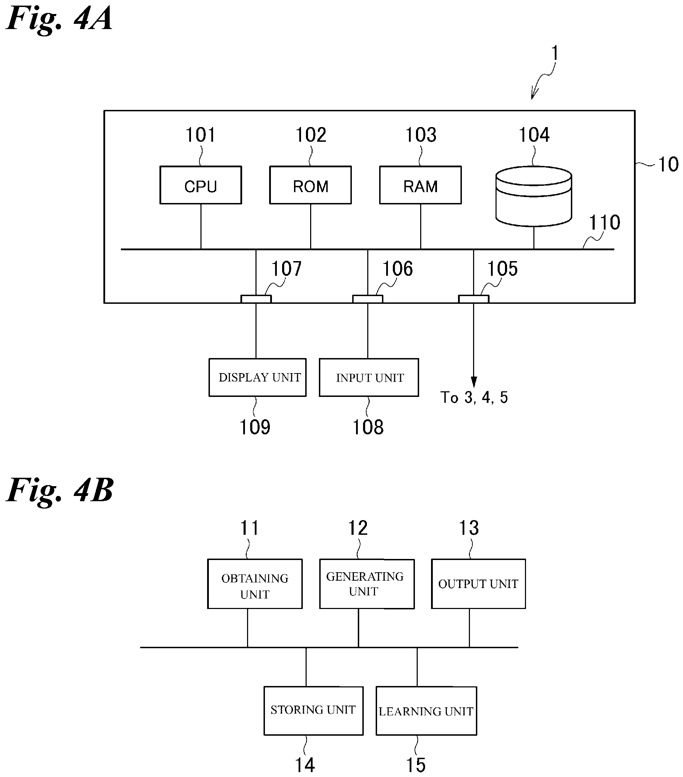

Fig. 4A is a schematic diagram illustrating an example of a configuration of the biometric information computing device 1 and Fig. 4B is a schematic diagram illustrating an example of a function of the biometric information computing device 1.

The biometric information computing device 1 includes, for example, as illustrated in Fig. 4A, a housing 10, a CPU (Central Processing Unit) 101, a ROM (Read Only Memory) 102, a RAM (Random Access Memory) 103, a storage unit 104, and I/Fs 105 to 107. Each of the configurations 101 to 107 is connected via an internal bus 110.

The CPU 101 controls the entire biometric information computing device 1. The ROM 102 stores operation codes of the CPU 101. The RAM 103 is a work area used in the operation of the CPU 101. The storage unit 104 stores various kinds of information such as the database and the evaluation data. As the storage unit 104, for example, a data storage device such as an SSD (Solid State Drive) or the like is used in addition to an HDD (Hard Disk Drive). For example, the biometric information computing device 1 may include a not illustrated GPU (Graphics Processing Unit).

The I/F 105 is an interface for transmitting and receiving various kinds of information with the server 4, the sensor 5, and the like via the communications network 3 as necessary. The I/F 106 is an interface for transmitting and receiving the information with an input unit 108. As the input unit 108, for example, a keyboard is used, and the user or the like of the biometric information computing device 1 inputs various kinds of information, control commands of the biometric information computing device 1, or the like via the input unit 108. The I/F 107 is an interface for transmitting and receiving various kinds of information with a display unit 109. The display unit 109 displays various kinds of information, the evaluation result, or the like stored in the storage unit 104. As the display unit 109, a display is used, and for example, in a case of a touch panel type, the display unit 109 is provided integrally with the input unit 108.

Fig. 4B is a schematic diagram illustrating an example of a function of the biometric information computing device 1. The biometric information computing device 1 includes an obtaining unit 11, a generating unit 12, an output unit 13, and a storing unit 14, and, for example, may include a learning unit 15. Each function illustrated in Fig. 4B is achieved by executing programs stored in the storage unit 104 or the like with the CPU 101 using the RAM 103 as a working area.

### <Obtaining Unit 11>

The obtaining unit 11 obtains the evaluation data based on a pulse wave of the user. For example, after obtaining the sensor data from the sensor 5 or the like, the obtaining unit 11 performs a process on the sensor data to obtain the evaluation data.

The obtaining unit 11 performs, for example, as illustrated in Fig. 3B, a filtering process (filter process) on the obtained sensor data. In the filter process, for example, a bandpass filter of 0.5 to 5.0 Hz is used. Thus, the obtaining unit 11 extracts data (pulse wave data) corresponding to the pulse wave of the user. The pulse wave data indicates, for example, data corresponding to the velocity pulse wave. The pulse wave data may indicate, for example, data corresponding to the acceleration pulse wave or the plethysmogram and can be appropriately set depending on the type and the usage of the sensor. The filter range of the bandpass filter can be appropriately set depending on the usage.

The obtaining unit 11 performs, for example, a differential process on the pulse wave data. For example, when the differential process is performed to the pulse wave data corresponding to the velocity pulse wave, the obtaining unit 11 obtains data (differential data) corresponding to the acceleration pulse wave. In the differential process, a two time differentiation may be performed in addition to a one time differentiation.

The obtaining unit 11 performs, for example, a dividing process on the differential data. In the dividing process, for example, differential data corresponding to the acceleration pulse wave of a plurality of cycles is divided into data (divided data) corresponding to the acceleration pulse waves of respective cycles. Therefore, for example, by performing the differential process on one differential data, the obtaining unit 11 can obtain a plurality of divided data. In the dividing process, the differential data can be divided for each of any cycles (for example, positive number multiple of cycle) depending on the usage.

For example, in the dividing process, the data amounts of the divided respective divided data are mutually different in some cases. In this case, the obtaining unit 11 may identify the divided data with the least data amount, and may perform reduction of the data amount (trimming) to the other divided data. This allows making the data amounts of the respective divided data uniform, thus facilitating data comparison between the respective divided data.

Additionally, for example, a normalization process may be performed to a value corresponding to a time axis of the divided data. In the normalization process, for example, the normalization in which the minimum value of the value corresponding to the time axis is set to 0 and the maximum value is set to 1 is performed. Accordingly, the data comparison between the respective divided data is facilitated.

The obtaining unit 11 may provide the divided data by, for example, calculating a mean of a plurality of divided data to which the reduction of the data amount or the normalization has been performed.

The obtaining unit 11 performs a normalization process on the divided data. In the normalization process, data that has been normalized (normalized data) is generated targeting values corresponding to the amplitudes. In the normalization process, for example, the normalization with which the lowest value of the amplitude is 0 and the highest value of the amplitude is 1 is performed. The obtaining unit 11 obtains, for example, the normalized data as the evaluation data. In this case, the data corresponding to the acceleration pulse wave of the user is obtained as the evaluation data.

Besides sequentially performing the respective processes described above, the obtaining unit 11 does not necessarily perform the differential process, for example, as illustrated in Fig. 3C. In this case, the data corresponding to the velocity pulse wave of the user is obtained as the evaluation data.

The obtaining unit 11 may perform, for example, only a part of the above-described processes. In this case, the obtaining unit 11 may obtain any of the pulse wave data, the differential data, the divided data, the trimmed divided data, and the divided data in which the value corresponding to the time axis has been normalized as the evaluation data, and the setting can be appropriately made depending on the usage.

### <Generating Unit 12>

The generating unit 12 refers to the database and generates the evaluation result including the feature of blood carbon dioxide (for example, the value of the blood carbon dioxide partial pressure) for the evaluation data. The generating unit 12 refers, for example, to the classification information stored in the database and calculates the feature of blood carbon dioxide relative to the evaluation data. The generating unit 12 uses, for example, a format for displaying preliminarily stored in the storage unit 104 or the like to generate the evaluation result converted into a format with which the user can understand the feature of blood carbon dioxide.

For example, when the classification information includes the trained model, the generating unit 12 may refer to the trained model and generate the evaluation result with a health condition result included. The health condition result indicates the health condition of the user associated with the evaluation data. In this case, the reference data used in generating the trained model includes state information indicating the health condition of the examinee from whom the training pulse wave has been measured. The state information includes information relating to details of the health condition of the examinee at the present and in the past, such as "healthy," "possibility of XXX," and "history of XXX disease in the past."

The plurality of training data used in generating the trained model may, for example, include only the input data and the reference data from when the examinee is healthy. In this case, in the evaluation result (the health condition result), the health condition of the user associated with the evaluation data may indicate only healthy or a state other than healthy. That is, the generating unit 12 may generate the evaluation result such that when the evaluation data is similar to the training data, "healthy" is indicated as the health condition result and when the evaluation data deviates from the training data, "a state other than healthy" is indicated as the health condition result. The threshold values that classify "healthy" and "a state other than healthy" can be appropriately set.

### <Output Unit 13>

The output unit 13 outputs the evaluation result. Besides outputting the evaluation result to a display unit 109, the output unit 13 may output the evaluation result to, for example, the sensor 5 or the like.

### <Storing Unit 14>

The storing unit 14 retrieves various kinds of data such as a database stored in the storage unit 104 as necessary. The storing unit 14 stores the various kinds of data obtained or generated by the configurations 11 to 13, and 15 in the storage unit 104 as necessary.

### <Learning Unit 15>

The learning unit 15 uses, for example, the plurality of training data and generates the classification information. The learning unit 15 may obtain, for example, new training data and update the existing classification information.

### <Communications Network 3>

The communications network 3 is a publicly known Internet network or the like in which the biometric information computing device 1, the server 4, and the sensor 5 are connected via a communication line. The communications network 3 may be established by a LAN (Local Area Network) or the like when the biometric information computing system 100 is operated in a certain narrow area. The communications network 3 may be established by what is called an optical fiber communications network. The communications network 3 is not limited to a wired communication network, may be achieved by a wireless communications network, and can be appropriately set depending on the usage.

In the server 4, the information transmitted via the communications network 3 is accumulated. The server 4 transmits the information accumulated via the communications network 3 to the biometric information computing device 1 based on a request from the biometric information computing device 1.

### <Sensor 5>

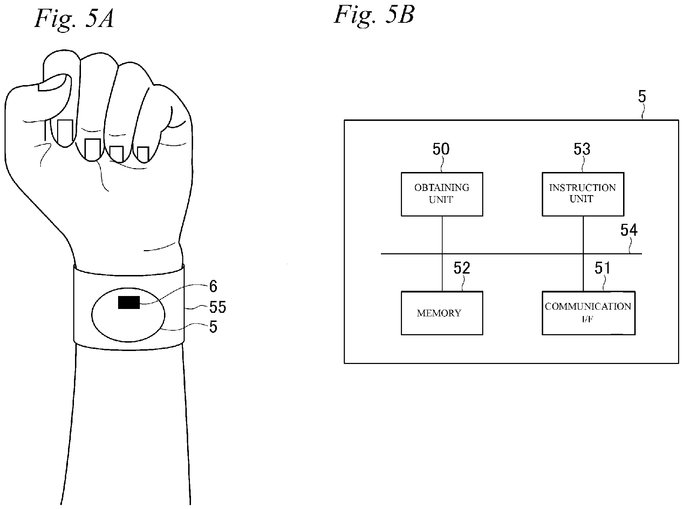

The sensor 5 generates the sensor data. The sensor 5 includes, for example, as illustrated in Fig. 5A, a detecting unit 6. The sensor 5 is worn on a position where the pulse wave of the user can be detected via the detecting unit 6 and is fixed to, for example, a wristband 55.

As the detecting unit 6, a publicly known detecting device that can detect the user's pulse wave is used. As the detecting unit 6, for example, at least any of a strain sensor such as a fiber bragg grating (FBG) sensor, a gyro sensor, one or more electrodes for measuring a pulse wave signal, a photoplethysmogram (PPG) sensor, a pressure sensor, and a photo-detection module is used. For example, a plurality of the detecting units 6 may be provided.

The sensor 5 may be embedded in clothing. The user wearing the sensor 5 may be not only a human but also a pet such as a dog and a cat, and for example, may be a domestic animal such as a cow and a pig, a farmed fish, or the like.

The sensor 5 includes, for example, as illustrated in Fig. 5B, an obtaining unit 50, a communication I/F 51, a memory 52, and an instruction unit 53, and each of the configurations is connected via an internal bus 54.

The obtaining unit 50 measures the user's pulse wave via the detecting unit 6, and generates the sensor data. The obtaining unit 50 transmits, for example, the generated sensor data to the communication I/F 51 or the memory 52.

The communication I/F 51 transmits the various kinds of data such as sensor data to the biometric information computing device 1 and the server 4 via the communications network 3. The communication I/F 51 includes a line control circuit for connecting to the communications network 3, a signal conversion circuit for performing a data communication with the biometric information computing device 1 and the server 4, and the like. The communication I/F 51 performs conversion processing to various kinds of instructions from the internal bus 54 and delivers these to the communications network 3 side, and when data from the communications network 3 is received, the communication I/F 51 performs predetermined conversion processing to the data, and transmits it to the internal bus 54.

The memory 52 stores various kinds of data such as sensor data transmitted from the obtaining unit 50. For example, the memory 52 receives an instruction from another terminal device connected via the communications network 3, thereby transmitting the stored various kinds of data such as sensor data to the communication I/F 51.

The instruction unit 53 includes an operating button, a keyboard, or the like for obtaining the sensor data, and for example, includes a processor, such as a CPU. When an instruction to obtain the sensor data is accepted, the instruction unit 53 notifies the obtaining unit 50 of it. The obtaining unit 50 that has received the notification obtains the sensor data. For example, as illustrated in Fig. 3B and Fig. 3C, the instruction unit 53 may perform a process for obtaining the evaluation data from the sensor data.

Here, as an example of obtaining the sensor data, a case of using an FBG sensor will be described.

The FBG sensor is one in which diffraction grating structures are formed at predetermined intervals in one optical fiber. The FBG sensor has a feature of, for example, a sensor part length of 10 mm, a wavelength resolution of ±0.1 pm, a wavelength range of 1550 ± 0.5 nm, a fiber diameter of 145 µm, and a core diameter of 10.5 µm. The measurement can be performed with the FBG sensor as the above-described detecting unit 6 in contact with the user's skin.

For example, as a light source used for the optical fiber, an ASE (Amplified Spontaneous Emission) light source with the wavelength range of 1525 to 1570 nm is used. An emitted light from the light source enters the FBG sensor via a circulator. A reflected light from the FBG sensor is guided to a Mach-Zehnder interferometer via the circulator, and an output light from the Mach-Zehnder interferometer is detected by an optical detector. The Mach-Zehnder interferometer is one for creating an interference light by splitting an optical path into two optical paths with an optical path difference by a beam splitter and superimposing the two optical paths into one again by the beam splitter. To provide the optical path difference, for example, the length of one optical fiber may be lengthened. Since interference fringes are produced in a coherent light corresponding to the optical path difference, by measuring the pattern of the interference fringe, the strain change in the FBG sensor, that is, the pulse wave can be detected. The obtaining unit 50 generates the sensor data based on the detected pulse wave. Accordingly, the sensor data is obtained.

The optical fiber sensor system that detects the waveform of the pulse wave by detecting the strain amount of the FBG sensor includes, in addition to the light source whose light is entered into the FBG sensor, means using an ASE light source with a wide bandwidth, an optical systems, such as a circulator, a Mach-Zehnder interferometer, and a beam splitter, a light receiving sensor included in the optical detector, and analysis means for analyzing a wavelength shift amount. The optical fiber sensor system can be used by selecting the light source and a bandwidth light depending on the characteristics of the used FBG sensor, and various kinds of methods can be employed also for the analysis means, such as a wave detection method.

### (Embodiment 1: Operation of Biometric Information Computing System 100)

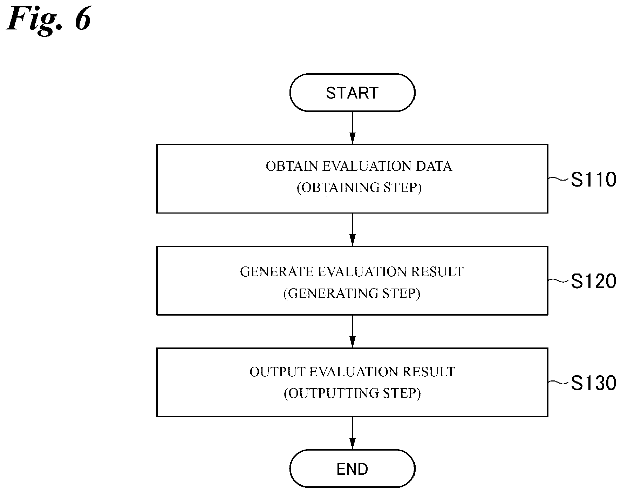

Next, an example of the operation of the biometric information computing system 100 according to the embodiment will be described. Fig. 6 is a flowchart illustrating an example of the operation of the biometric information computing system 100 in this embodiment.

The biometric information computing system 100 is executed, for example, via a biometric information computing program installed in the biometric information computing device 1. That is, the user can operate the biometric information computing device 1 or the sensor 5 to obtain the evaluation result including the feature of blood carbon dioxide (for example, the value of the blood carbon dioxide partial pressure) from the sensor data through the biometric information computing program installed in the biometric information computing device 1.

The operation of the biometric information computing system 100 includes obtaining means S110 and generating means S120, and may include, for example, output means S130.

### <Obtaining Means S 110>

The obtaining means S110 obtains evaluation data based on the pulse wave of the user. For example, the obtaining unit 50 of the sensor 5 measures the pulse wave of the user via the detecting unit 6 to generate the sensor data. The obtaining unit 50 transmits the sensor data to the biometric information computing device 1 via the communication I/F 51 and the communications network 3. The obtaining unit 11 of the biometric information computing device 1 receives the sensor data from the sensor 5.

The obtaining unit 11 performs, for example, the process illustrated in Fig. 3B on the sensor data, thereby obtaining the evaluation data. The obtaining unit 11 stores the obtained evaluation data in the storage unit 104 via, for example, the storing unit 14. The conditions, such as a frequency of the obtaining unit 11 obtaining the sensor data from the sensor 5, can be appropriately set depending on the usage.

### <Generating Means S120>

Next, the generating means S120 refers to the database and generates the evaluation result including the feature of blood carbon dioxide (for example, the value of the blood carbon dioxide partial pressure) for the evaluation data. For example, the generating unit 12 refers to the classification information and calculates the value of the blood carbon dioxide partial pressure relative to the evaluation data. The generating unit 12 generates the evaluation result including the calculated value. The generating unit 12 stores the generated evaluation result in the storage unit 104 via, for example, the storing unit 14. For the value of the blood carbon dioxide partial pressure, besides a specific value being indicated, for example, an error range (for example, "XX ±2 mmHg") may be calculated.

The generating unit 12 may calculate respective values of the blood carbon dioxide partial pressure relative to the plurality of evaluation data with, for example, different measurement periods and makes the evaluation result include the respective values in. In this case, the user or the like can easily obtain a secular change, an average value, and the like of the blood carbon dioxide partial pressure.

### <Output Means S130>

Next, for example, the output means S 130 may output the evaluation result. For example, the output unit 13 outputs the evaluation result to the display unit 109.

Accordingly, the operation of the biometric information computing system 100 ends. The frequency and the order of executing the steps can be appropriately set depending on the usage.

According to the embodiment, the generating means S 120 refers to the database and generates the evaluation result including the feature of blood carbon dioxide for the evaluation data. The database stores the classification information calculated using the plurality of training data. Therefore, when the evaluation result is generated, it is possible for the evaluation result to include the quantitative feature of blood carbon dioxide in the light of the data that has resulted in the past (the input data and the feature of blood carbon dioxide). This allows attempting improvement in evaluation accuracy.

According to the embodiment, the generating means S 120 can refer to the trained model and generate the evaluation result with including the health condition result associated with the evaluation data. Therefore, the user can be notified of the health condition determined by the feature of blood carbon dioxide, and the health condition can be obtained easily compared with the case where the user is notified only of the feature of blood carbon dioxide. This allows attempting improvement in convenience for the user.

According to the embodiment, the plurality of training data includes only the input data and the reference data from when the examinee is healthy and the health condition result indicates if the health condition of the user associated with the evaluation data is healthy or not. Therefore, even when the input data based on the training pulse wave when it is unhealthy and the feature of blood carbon dioxide cannot be prepared, the result indicating the health condition of the user can be generated. This allows attempting further improvement in convenience for the user without increasing the degree of difficulty when the classification information is generated.

According to the embodiment, the classification information is a calibration model obtained by using the PLS regression analysis with the input data as the explanatory variable and the reference data as the objective variable. Therefore, compared with the case where the classification information is calculated using machine learning or the like, the number of the training data can be significantly reduced and the calibration model can be easily updated. This allows establishing the biometric information computing system 100 and attempting to facilitate the update.

### (Second Embodiment: Biometric Information Computing System 100)

Next, an example of a biometric information computing system 100 according to a second embodiment will be described. The difference between the above-described embodiment and the second embodiment is that preliminary evaluation data based on the pulse wave is obtained. For the contents similar to the above-described embodiment, the explanation will be omitted.

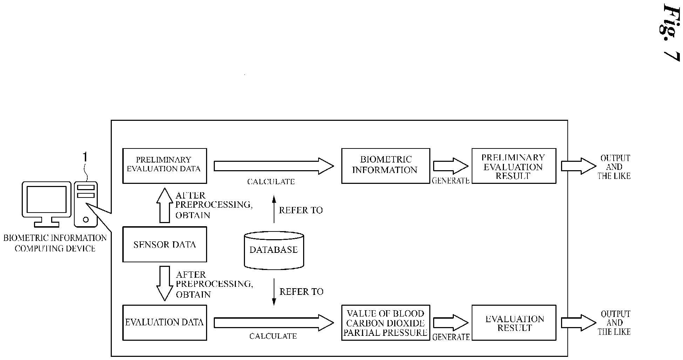

The biometric information computing device 1 according to the embodiment performs two types of processes on one sensor data generated based on the pulse wave, for example, as illustrated in Fig. 7. Thus, the biometric information computing device 1 obtains evaluation data and the preliminary evaluation data different from the evaluation data. A plurality of the preliminary evaluation data may be obtained.

The biometric information computing device 1 calculates biometric information for the preliminary evaluation data and generates, for example, a preliminary evaluation result including the biometric information. For example, the biometric information computing device 1 can output the preliminary evaluation result in addition to the evaluation result. For example, the biometric information computing device 1 may make the evaluation result include the biometric information.

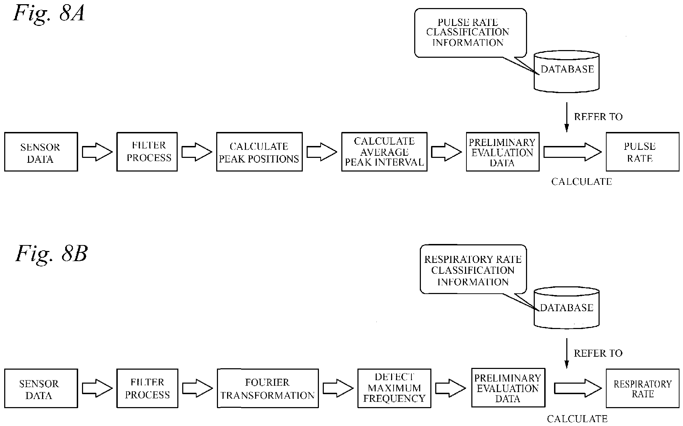

For the biometric information, for example, at least any one of a pulse rate and a respiratory rate can be calculated. In this case, for example, as illustrated in Fig. 8A and Fig. 8B, the content of the preprocessing performed at the obtaining means S 110 and the content of the database referred to at the generating means S 120 may differ. The following describes an example where the pulse rate and the respiratory rate are calculated as the biometric information.

### <Pulse Rate Calculation>

For example, when the pulse rate is calculated, as illustrated in Fig. 8A, the obtaining unit 11 performs the above-described filter process on the sensor data and extracts the pulse wave data.

The obtaining unit 11 performs a peak position calculating process on the pulse wave data. In the peak position calculating process, a plurality of peaks (maximum values of amplitudes) included in the pulse wave data are detected and the order of being sampled (corresponding to the time since the start of measurement) is identified. Thus, the obtaining unit 11 obtains peak position data included in the pulse wave data.

Then, the obtaining unit 11 performs an average peak interval calculating process on the peak position data. The average peak interval calculating process calculates peak intervals (the difference between the adjacent orders in which the peaks are sampled) included in the peak position data to calculate, for example, an average value of the peak intervals. Then, the obtaining unit 11 divides the peak intervals or the average value of the peak intervals by a sampling rate of the sensor data and obtains the data indicating the peak intervals corresponding to seconds as the preliminary evaluation data.

Then, the generating unit 12 refers to the database and calculates the pulse rate relative to the preliminary evaluation data. In this respect, the generating unit 12 refers to pulse rate classification information stored in the database. The pulse rate classification information indicates a function that divides, for example, 60 [seconds] by the peak intervals. Therefore, the generating unit 12 can calculate, for example, the pulse rate (= 71 [bpm]) relative to the preliminary evaluation data (the peak interval = 0.85 [seconds]). This allows the generating unit 12 to generate the evaluation result or the preliminary evaluation result including the pulse rate.

### <Respiratory Rate Calculation>

For example, when the respiratory rate is calculated, as illustrated in Fig. 8B, the obtaining unit 11 performs the above-described filter process on the sensor data to extract the pulse wave data.

Then, the obtaining unit 11 performs Fourier transform processing on the pulse wave data. In the Fourier transform processing, for example, the pulse wave data indicating sampling period to amplitude is converted into frequency data indicating frequency to intensity. This causes the obtaining unit 11 to obtain the frequency data relative to the pulse wave data.

Then, the obtaining unit 11 performs a maximum frequency detecting process on the frequency data. In the maximum frequency detecting process, the frequency of the maximum intensity between 0.15 Hz to 0.35 Hz in the frequency data is identified. This causes the obtaining unit 11 to obtain the value of the identified frequency as the preliminary evaluation data.

Then, the generating unit 12 refers to the database and calculates the respiratory rate relative to the preliminary evaluation data. In this respect, the generating unit 12 refers to respiratory rate classification information stored in the database. The respiratory rate classification information indicates, for example, a function that multiplies the identified frequency by 60 [second]. Therefore, the generating unit 12 can calculate, for example, the respiratory rate (= 13.5 [bpm]) relative to the preliminary evaluation data (the identified frequency = 0.225 Hz). Thus, the generating unit 12 can generate, for example, the evaluation result or the preliminary evaluation result including the respiratory rate.

Thus, the biometric information computing device 1 can set the preprocessing on the sensor data depending on the content of the biometric information included in the preliminary evaluation result and appropriately set the content of the database to be referred to.

For the biometric information, besides the above-described pulse rate and respiratory rate, for example, information calculatable based on the pulse wave, such as a stress, a blood glucose level, a blood pressure, an oxygen saturation, a vascular age, and a degree of diabetes can be used.

Corresponding to the content of the biometric information to be calculated, the preliminary classification information used for the calculation may be stored in the database in advance. In this case, a plurality of pairs of preliminary input data indicating the training pulse wave described above and preliminary reference data including the biometric information associated with the preliminary input data are prepared as preliminary training data. Then, for example, the learning unit 15 generates the preliminary classification information using the plurality of preliminary training data and stores it in the database.

The obtainment of the biometric information included in the preliminary reference data can be performed by measuring the examinee using a measuring device for measuring necessary biometric information in a similar way to, for example, that of the above-described reference data. For a method for learning the preliminary classification information, a method similar to that of the above-described classification information can be used.

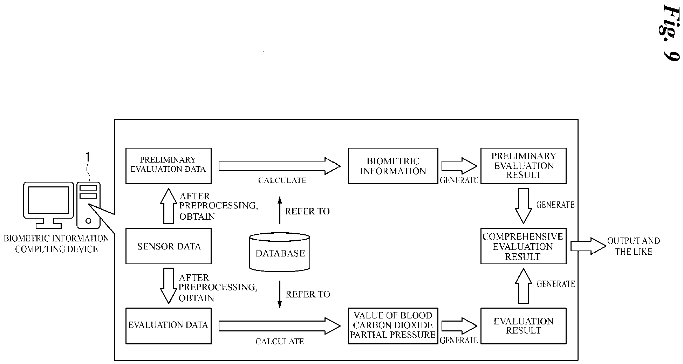

Besides what is described above, the biometric information computing device 1 can, for example, as illustrated in Fig. 9, generate a comprehensive evaluation result based on the evaluation result and the preliminary evaluation result and, for example, output it. Therefore, the biometric information computing system 100 can use the preliminary evaluation result generated by a different perspective from the evaluation result, thereby ensuring multifaceted evaluations.

The biometric information computing device 1 performs the two types of processes illustrated in for example, Fig. 3B and Fig. 3C, and thus, the evaluation data and the preliminary evaluation data may be obtained from the sensor data. That is, when the data corresponding to the acceleration pulse wave of the user is obtained as the evaluation data, the data corresponding to the velocity pulse wave of the user is obtained as the preliminary evaluation data. In this case, by using the evaluation result generated for the evaluation data and the preliminary evaluation result generated for the preliminary evaluation result, an evaluation with higher accuracy can be achieved.

According to the embodiment, in addition to the effect of the embodiment described above, the generating means S120 refers to the database and generates the preliminary evaluation result including the biometric information of the user for the preliminary evaluation data. Therefore, the preliminary evaluation result generated by a different perspective from the evaluation result can be used, thereby ensuring the multifaceted evaluations. Thus, an appropriate evaluation corresponding to the request by the user can be achieved.

According to the embodiment, the generating means S120 includes referring to the preliminary classification information and generating the preliminary evaluation result for the preliminary evaluation data. Therefore, the preliminary evaluation result generated by referring to the information different from the evaluation result can be used, thereby ensuring more multifaceted evaluations. Thus, an appropriate evaluation corresponding to the request by the user can be easily achieved.

### (Third Embodiment: Biometric Information Computing System 100)

Next, an example of a biometric information computing system 100 in the third embodiment will be described. The difference between the above-described embodiments and the third embodiment is that classification information appropriate for the evaluation data is selected from a plurality of pieces of the classification information. For the contents similar to the above-described embodiments, the explanation will be omitted.

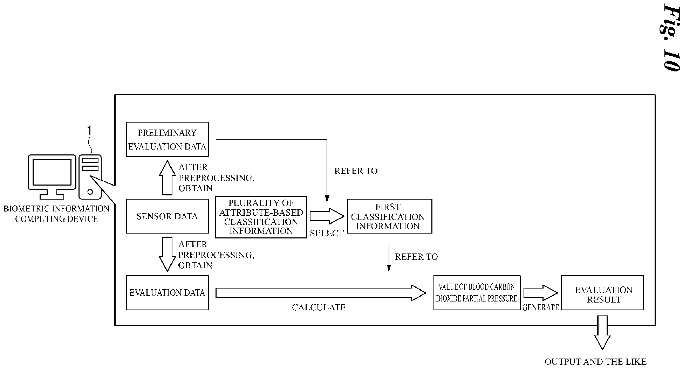

The biometric information computing device 1 according to the embodiment, for example, as illustrated in Fig. 10, refers to preliminary evaluation data and selects specific classification information (for example, first classification information) among a plurality of pieces of attribute-based classification information (selecting means). The biometric information computing device 1 refers to the selected first classification information and calculates the feature of blood carbon dioxide (for example, the value of the blood carbon dioxide partial pressure) relative to the evaluation data to generate the evaluation result (attribute-based generating means).

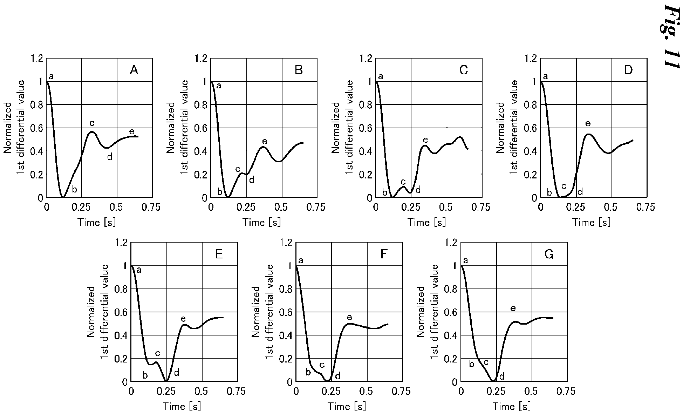

The plurality of pieces of attribute-based classification information are calculated using mutually different training data. For example, when data corresponding to the acceleration pulse wave of the examinee is used as the input data of the training data, for example, the input data is prepared for each of seven types (A to G) as illustrated in Fig. 11, thus generating seven types of attribute classification information.

When such plurality of pieces of attribute-based classification information are stored in the database, for example, the obtaining unit 11 obtains the evaluation data corresponding to the acceleration pulse wave of the user and the preliminary evaluation data. The generating unit 12 then refers to the preliminary evaluation data and selects the first classification information. Afterwards, the generating unit 12 refers to the first classification information and calculates the feature of blood carbon dioxide (for example, the value of the blood carbon dioxide partial pressure) relative to the evaluation data. Therefore, among the respective pieces of the attribute classification information, the optimal classification information for the user can be selected.

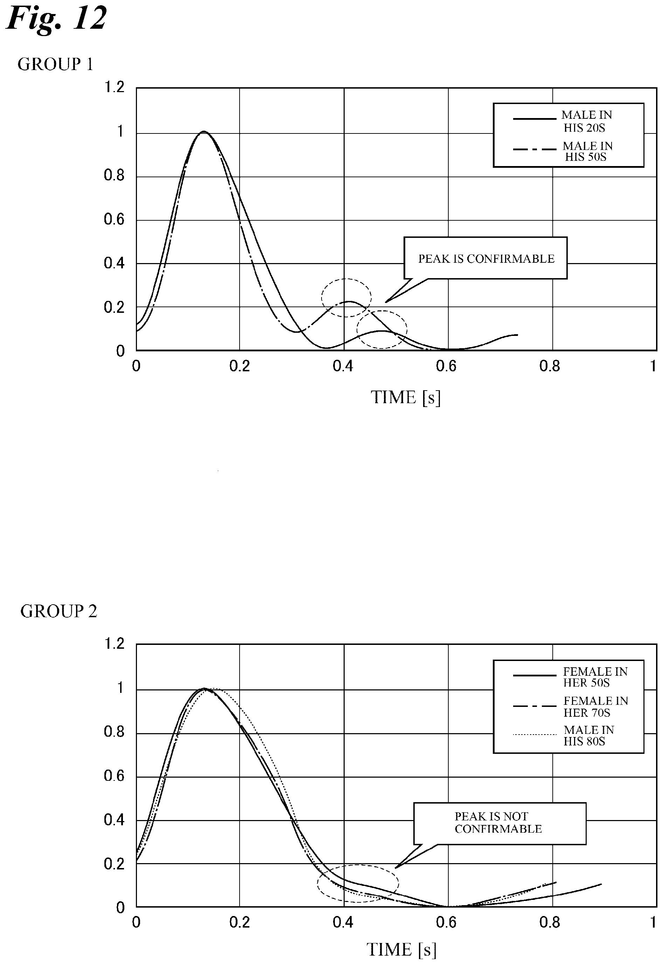

For example, when data corresponding to the velocity pulse wave of the examinee is used as the input data of the training data, for example, the input data may be prepared for each of two types (group 1, group 2) as illustrated in Fig. 12 to generate two types of attribute classification information.

Here, the data corresponding to the acceleration pulse wave illustrated in Fig. 11 is easily classified into details based on the feature, whereas when the feature of blood carbon dioxide is calculated, it is concerned that the accuracy deteriorates in association with the false detection of the peaks and the like. While the data corresponding to the velocity pulse wave illustrated in Fig. 12 is difficult to be classified into details based on the feature compared with the data corresponding to the acceleration pulse wave, the feature of blood carbon dioxide can be calculated with high accuracy since the false detection of the peaks and the like is less.

In the light of what is described above, the plurality of pieces of attribute classification information may include, for example, the data corresponding to the acceleration pulse wave as illustrated in Fig. 11 as the selection data used for selecting the specific classification information and for the training data when the attribute classification information is generated, the data corresponding to the velocity pulse wave may be used.

In this case, as the obtaining means S 110, for example, the obtaining unit 11 obtains data corresponding to the velocity pulse wave from the sensor data based on the pulse wave of the user as the evaluation data. The obtaining unit 11 obtains data corresponding to the acceleration pulse wave from the sensor data as the preliminary evaluation data.

Next, as the selecting means, for example, the generating unit 12 refers to the preliminary evaluation data and identifies selection data (first selection data) the most similar to the preliminary evaluation data among a plurality of selection data including the data corresponding to the acceleration pulse wave to select first classification information associated with the first selection data. As the attribute-based generating means, the generating unit 12 refers to the first classification information and generates the evaluation result for the evaluation data. This allows attempting the further improvement of the evaluation accuracy.

Here, an example of the data used for the above-described selection data or the like will be described.

For example, as illustrated in Fig. 11, there are inflection points of ***a*** to ***e*** in the acceleration pulse wave. For example, when the point ***a*** is the maximum peak in the acceleration pulse wave and the respective inflection points in the order from the point ***a*** are the point ***b***, the point ***c***, the point ***d***, and the point ***e***, and the normalization is performed with the point ***a*** as 1 and the point ***b*** or the point ***d***, which are the minimum values, as 0, the acceleration pulse wave can be classified into seven patterns using a method of classifying by the values of the respective inflection points and a magnitude relationship of the differences of the values. First, when the values of the inflection points satisfy ***b*** < ***d***, it is classified into a pattern A or B. When ***b*** < ***d*** and, furthermore, c ≥ 0.5 are satisfied, it is classified into A, or otherwise into B. Next, when the values of the inflection points satisfy ***b*** ≈ ***d**,* it is classified into a pattern C or D. When ***b*** ≈ ***d*** and, furthermore, ***c*** ≈ 0 are satisfied, it is classified into the pattern D, or otherwise into the pattern C. Finally, when ***b*** > ***d*** is satisfied, it can be classified into any one of a pattern E, F, or G. It is classified into the pattern E when ***b*** > ***d*** and, furthermore, ***b*** < ***c*** are satisfied, into the pattern F when ***b*** ≈ ***c*** is satisfied, and into the pattern G when ***b*** > ***c*** is satisfied.

For example, the generating unit 12 determines, for example, into which pattern in Fig. 11 the preliminary evaluation data fits and identifies the first selection data. For example, when the input inflection point ***b*** of the preliminary evaluation data is smaller than the inflection point ***d*** and, furthermore, the inflection point ***c*** ≥ 0.5 is satisfied, the pattern A is the first selection data. Thus, the classification information appropriate for the feature of the evaluation data is referred to, thereby ensuring an accurate calculation of the feature of blood carbon dioxide.

According to the embodiment, in addition to the effects of the above-described embodiments, the generating means S 120 includes the selecting means that refers to the preliminary evaluation data and selects the first classification information, and the attribute-based generating means that refers to the first classification information and generates the evaluation result for the evaluation data. Therefore, the optimal attribute classification information to the feature of the pulse wave is selected, and then, the evaluation result for the evaluation data can be generated. This allows attempting the further improvement of the evaluation accuracy.

According to the embodiment, the obtaining means S110 obtains the data corresponding to the velocity pulse wave based on the pulse wave as the evaluation data. The obtaining means obtains the data corresponding to the acceleration pulse wave based on the pulse wave as the preliminary evaluation data. Therefore, the attribute classification information can be selected using the acceleration pulse wave that facilitates the classification of the feature of the pulse wave compared with the velocity pulse wave. The evaluation result can also be generated using the velocity pulse wave that facilitates the calculation of the feature of blood carbon dioxide compared with the acceleration pulse wave. This allows attempting the further improvement of the evaluation accuracy.

While in the respective embodiments described above, the case where the value of the blood carbon dioxide partial pressure is mainly used as the feature of blood carbon dioxide has been described, the same applies to the case where at least any one of the dissolved concentration of blood carbon dioxide, the concentration of bicarbonate contained in blood, and pH of blood is used, thereby omitting the description. In particular, when the value of the blood carbon dioxide partial pressure is used as the feature of blood carbon dioxide, the reference data tends to be easily collected compared with the other values. Therefore, it is possible to achieve a significant reduction in time and cost spent when the classification information is generated.

While the embodiments of the present invention have been described, the embodiments have been presented as examples, and are not intended to limit the scope of the invention. The novel embodiments described herein can be embodied in a variety of other configurations. Various omissions, substitutions and changes can be made without departing from the gist of the invention. The embodiments and the modifications thereof are within the scope and the gist of the invention and within the scope of the inventions described in the claims and their equivalents.

### DESCRIPTION OF REFERENCE SIGNS

- 1:: Biometric information computing device
- 3:: Communications network
- 4:: Server
- 5:: Sensor
- 6:: Detecting unit
- 10:: Housing
- 11:: Obtaining unit
- 12:: Generating unit
- 13:: Output unit
- 14:: Storing unit
- 15:: Learning unit
- 50:: Obtaining unit
- 51:: Communication I/F
- 52:: Memory
- 53:: Instruction unit
- 54:: Internal bus
- 55:: Wristband
- 100:: Biometric information computing system
- 101:: CPU
- 102:: ROM
- 103:: RAM
- 104:: Storage unit
- 105:: I/F
- 106:: I/F
- 107:: I/F
- 108:: Input unit
- 109:: Display unit
- 110:: Internal bus
- S110:: Obtaining means
- S120:: Generating means
- S13 0:: Outputting means

## Claims

1. A biometric information computing system for outputting information relating to a feature of blood carbon dioxide of a user comprising:
obtaining means that obtains evaluation data based on a pulse wave of the user;
a database that stores classification information generated using a plurality of training data, the training data being a pair of input data based on a preliminarily obtained training pulse wave and reference data including the feature of blood carbon dioxide associated with the input data; and
generating means that refers to the database and generates an evaluation result including the feature of blood carbon dioxide for the evaluation data.

2. The biometric information computing system according to claim 1, wherein
the reference data includes state information indicating a health condition of an examinee from whom the training pulse wave has been measured,
the classification information includes a trained model generated by machine learning using a plurality of the training data, and
the generating means includes referring to the trained model and generating the evaluation result with a health condition result included, the health condition result indicating a health condition of the user associated with the evaluation data.

3. The biometric information computing system according to claim 2, wherein
the plurality of training data includes only the input data and the reference data from when the examinee is healthy, and
the health condition result indicates that the health condition of the user associated with the evaluation data is healthy or a state other than healthy.

4. The biometric information computing system according to claim 1, wherein
the classification information is a calibration model obtained using a PLS regression analysis with the input data as an explanatory variable and the reference data as an objective variable.

5. The biometric information computing system according to claim 1, wherein
the obtaining means includes obtaining preliminary evaluation data different from the evaluation data based on the pulse wave, and
the generating means includes referring to the database and calculating biometric information of the user for the preliminary evaluation data.

6. The biometric information computing system according to claim 5, wherein
the database stores preliminary classification information generated using a plurality of preliminary training data, the preliminary training data being a pair of preliminary input data based on the training pulse wave and preliminary reference data including the biometric information associated with the preliminary input data, and
the generating means includes referring to the preliminary classification information and calculating the biometric information for the preliminary evaluation data.

7. The biometric information computing system according to claim 1, wherein
the obtaining means includes obtaining preliminary evaluation data different from the evaluation data based on the pulse wave,
the classification information includes a plurality of pieces of attribute-based classification information calculated using the training data different from one another, and
the generating means includes:
selecting means that refers to the preliminary evaluation data and selects first classification information among the plurality of pieces of attribute-based classification information; and
attribute-based generating means that refers to the first classification information and generates the evaluation result for the evaluation data.

8. The biometric information computing system according to claim 7, wherein
the obtaining means includes:
obtaining data corresponding to a velocity pulse wave based on the pulse wave as the evaluation data; and
obtaining data corresponding to an acceleration pulse wave based on the pulse wave as the preliminary evaluation data.
